# EUROPÄISCHE PATENTSCHRIFT

(11) **EP 0 512 348 B1**
(45) Veröffentlichungstag und Bekanntmachung des Hinweises auf die Patenterteilung: **27.12.1995**
(21) Anmeldenummer: 92107129.6
(22) Anmeldetag: 27.04.1992
(51) Int. Cl.: C07D 313/00

(54) **Derivate cyclischer Lactone, Verfahren zu ihrer Herstellung und Verfahren zur Herstellung von 15-Pentadecanolid und seinen Homologen**
Cyclic lactone derivatives, process for their preparation and process for the preparation of 15-pentadecanolide and their homologs
Dérivés de lactone cyclique, procédé pour leur préparation et procédé de préparation de 15-pentadécanolide et ses homologues

(30) Priorität: 09.05.1991 DE 4115182
(43) Veröffentlichungstag der Anmeldung: 11.11.1992
(73) Patentinhaber: HAARMANN & REIMER GMBH, D-37601 Holzminden (DE)
(72) Erfinder: Hopp, Rudolf, Dr., W-3450 Holzminden (DE); Finkelmeier, Horst, Dr., W-3450 Holzminden (DE); Koch, Oskar, Dr., W-3400 Göttingen (DE); Körber, Alfred, Dr., W-3450 Holzminden (DE)
(74) Vertreter: Petrovicki, Wolfgang, Dr.

(56) Entgegenhaltungen:
- CHEMICAL ABSTRACTS, vol. 102, no. 27, 1985, Columbus, Ohio, US; abstract no. 203884P, Seite 591, Spalte 2 .
- Advanced Organic Chemistry. J March. 3rd Edn., 1985, Seiten 901, 902, 905.
- Ibid, Seiten 898-899

## Beschreibung

Die Erfindung betrifft neue Derivate cyclischer Lactone, die sich zur Herstellung von 15-Pentadec-11/12-enolid und seinen Homologen, Ausgangsprodukten zur Herstellung von 15-Pentadecanolid (= 15-Hydroxy-pentadecansäurelacton) und seinen Homologen eignen, ein Verfahren zur Herstellung dieser neuen Derivate sowie ein Verfahren zur Herstellung von 15-Pentadecanolid und seinen Homologen, das von diesen neuen Derivaten ausgeht.

Moschus ist bekanntlich selten und teuer. Deshalb sind Duftstoffe mit moschusähnlichem Geruch, die besser zugänglich sind, begehrte Komponenten für die Duftstoffindustrie. 15-Pentadecanolid der Formel 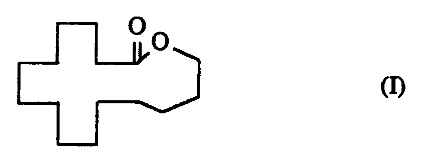
ist Bestandteil des Angelicawurzelöls und besitzt - wie seine unmittelbaren Homologen - einen zarten moschusartigen Geruch und die Fähigkeit als Fixativ zu wirken. Man hat sich deswegen schon intensiv mit der Herstellung solcher makrocyclischen Lactone befaßt. Die heute wichtigsten Synthesen gehen von 13-Oxabicyclo[10.4.0]hexadec-1(12)en II aus, das z.B. durch radikalische Addition von Allylalkohol an Cyclododecanon und säurekatalysierte Dehydratisierung des resultierenden 2-(γ-Hydroxypropyl)-cyclododecanons hergestellt werden kann (DE-AS 2 136 496): 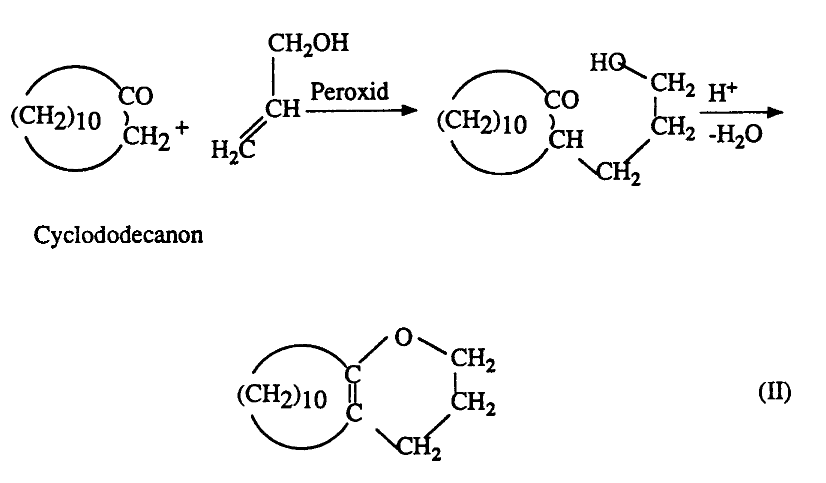
Nach einem anderen Verfahren addiert man Wasserstoffperoxid oder Alkylperoxid an II in Gegenwart von Schwefelsäure. Thermische oder UV-initiierte Spaltung des entstandenen 12-Hydroperoxy-13-oxabicyclo[10.4.0]hexadecans (III) führt zu 15-Pentadecanolid (I) und zu 15-Pentadecenoliden, die zu I hydriert werden können (DE-AS 2 026 056): 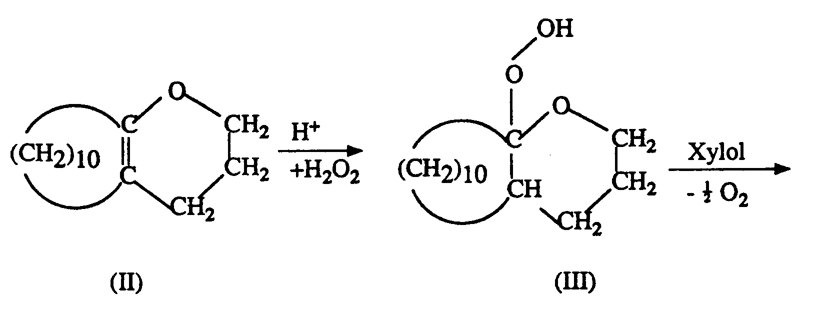 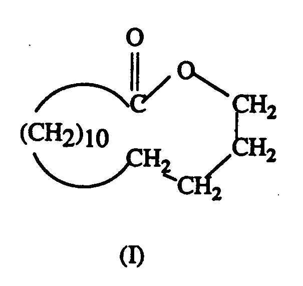
Ein weiteres Verfahren macht sich das aus II durch Nitrosierung zugängliche 12-Oximino-15-pentadecanolid (IV), das entsprechende 12-Keton oder das 12-Hydrazon zunutze; durch Reduktion nach Wolff-Kishner oder Huang-Minlon erhält man 15-Hydroxy-pentadecansäure (V) 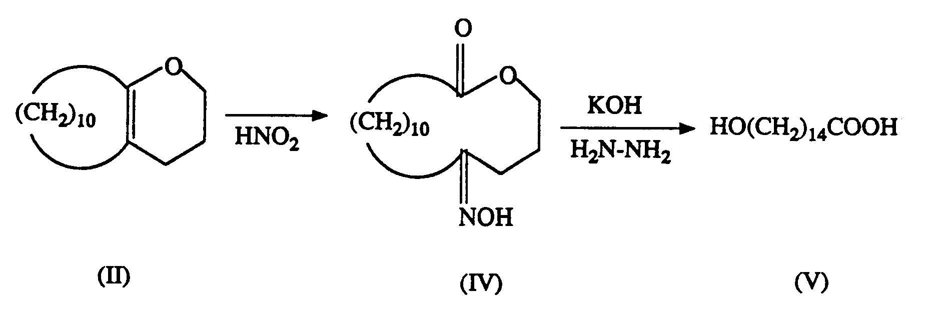
die sich ihrerseits zu I cyclisieren läßt, wobei sie vorzugsweise zunächst zum Polyester kondensiert wird der durch Vakuumdestillation analog W.H. Carothers, J. Amer. Chem. Soc. 58, 654 (1936) zu I depolymerisiert (DE-OS 2 731 543) werden kann.

Das Verfahren des russischen Urheberscheins 521 274 stellt eine Variante dar, bei welcher der Makrocyclus in allen Reaktionsschritten erhalten bleibt: II wird mit Butylnitrit/Natriumhydrogensulfit in das 12-Oxo-15-pentadecanolid (VI) überführt und dieses nach Clemmensen zum 15-Pentadecanolid reduziert: 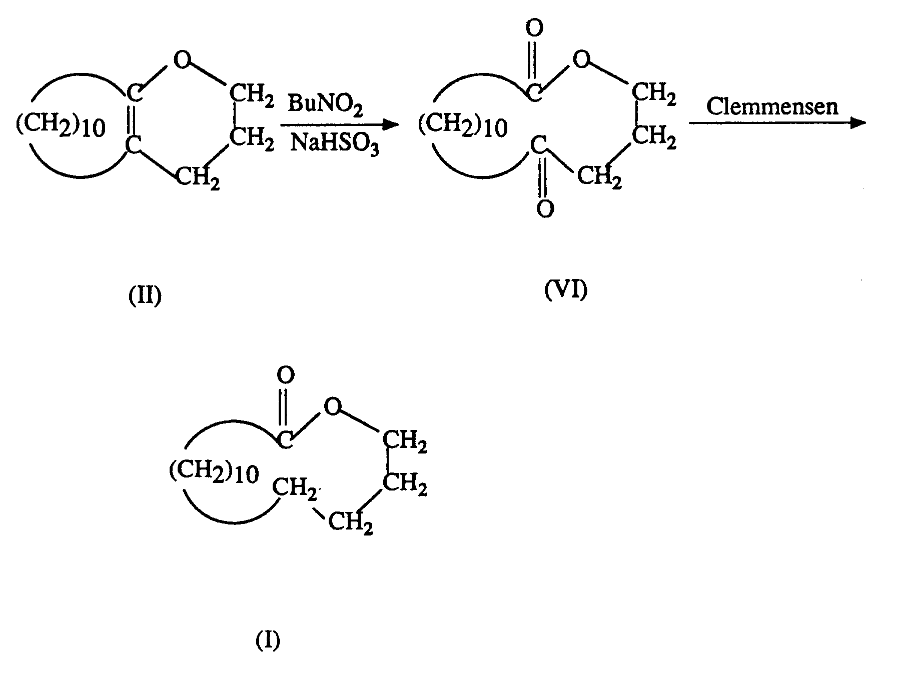
Die Ausbeute an I beträgt bei diesem Verfahren nur 36 %, wie aus den Beispielen hervorgeht. Auch die elektrochemische Reduktion von VI nach I gemäß DE-OS 3 127 242 verläuft in nur mäßigen Ausbeuten von ca. 60 %.

Die Erhaltung des Makrocyclus während des Reduktionsgeschehens wird weiterhin in dem brasilianischen Patent 81 08 358 beschrieben, wobei das Tosylhydrazon von VI durch teure Borhydride (z.B. Na-cyanoborhydrid) zu I reduziert wird.

Gemäß dem russischen Urheberschein 1 133 274 wird das 12-Keton VI in Gegenwart von Raney-Nickel zum 12-Hydroxy-15-pentadecanolid (VII) reduziert, dieses anschließend beispielsweise in Gegenwart von Phosphorsäure zu den entsprechenden 15-Pentadec-11- und -12-enoliden (VIII) dehydratisiert und diese Produkte in Gegenwart eines Nickelkatalysators zu I hydriert: 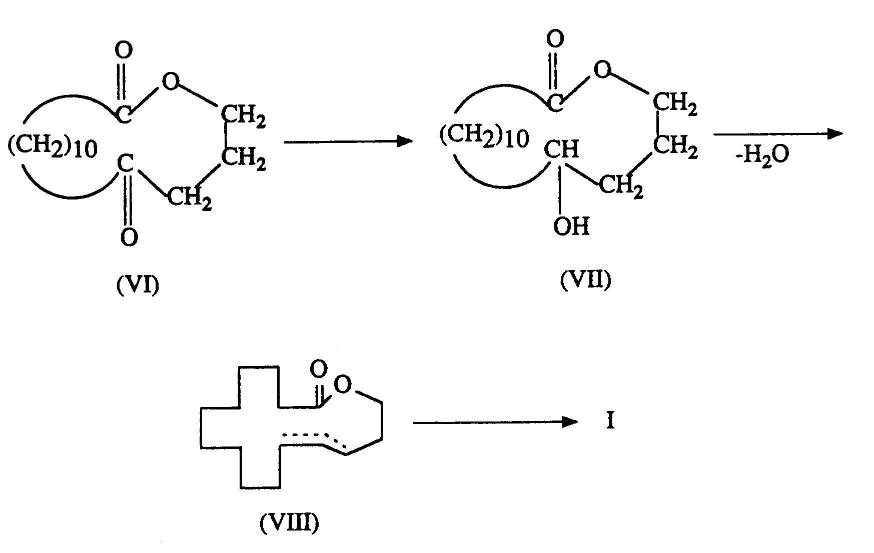
Die im Urheberschein mit 75 bis 78 % angegebenen Ausbeuten reduzieren sich nach unseren Erfahrungen bei größeren Ansätzen auf 50 bis 60 %.

Sämtliche diskutierten Verfahren des Standes der Technik leiden an mindestens einem für die industrielle Produktion wesentlichen Mangel. Cyclisierungen werden nach dem Verdünnungsprinzip durchgeführt und erfordern deshalb unverhälmismäßig große Lösungsmittelmengen; bei der Depolymerisation von Polyestern entsteht ein hoher Ausbeuteverlust durch nicht aufarbeitbaren Rückstand. Man hatte - vereinfacht gesagt - bis jetzt die Wahl zwischen einfacheren Verfahren mit schlechter Ausbeute und komplizierten Verfahren mit besserer Ausbeute. Ein elegantes Verfahren mit guter Ausbeute war bislang unbekannt.

Überraschenderweise wurde nun gefunden, daß sich die Hydroxylactone 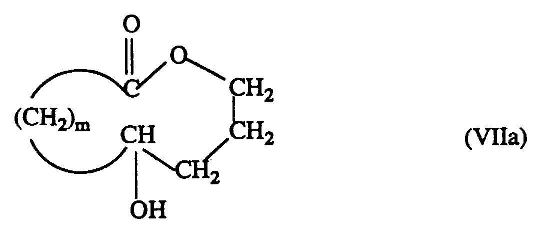
m = 9, 10 oder 11
z.B. 12-Hydroxy-15-pentadecanolid (m = 10) mit geeigneten Reagenzien zu entsprechenden Derivaten IX umsetzen läßt, die außergewöhnlich glatt und in hoher Ausbeute zu den ungesättigten Lactonen 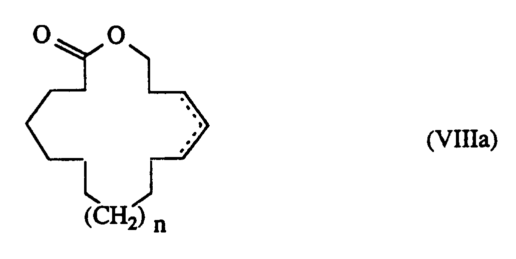
n = Null, 1 oder 2
pyrolisiert werden können, die man wiederum zu den gesättigten Lactonen 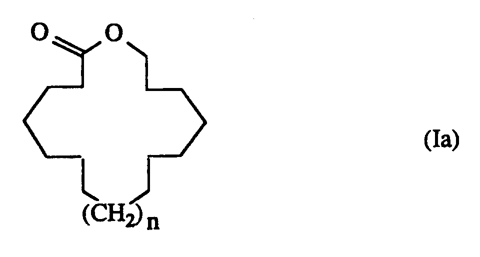
n = Null, 1 oder 2
hydrieren kann.

Dies ist insofern besonders unerwartet, als makrocyclische Lactone unter Pyrolysebedingungen lt. Literatur, z.B. J. Org. Chem. 42, 3895 (1977), unter Ringöffnung in hohen Ausbeuten zu ω-Alkensäuren gespalten werden. Weiterhin wurde gefunden, daß diese Reaktion auch auf die entsprechenden Homologen anwendbar ist.

Gegenstand der Erfindung sind also Lactone der Formel 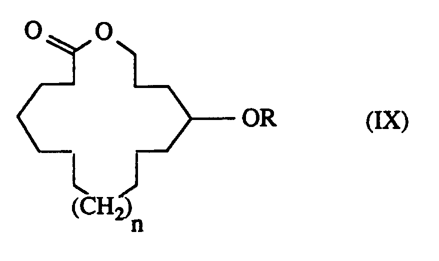
worin
n Null, 1 oder 2 bedeutet und
OR aus der Reihe bestehend aus 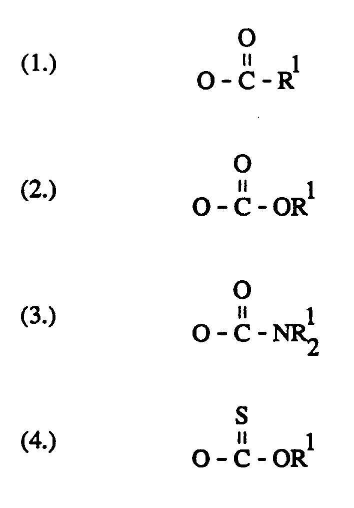 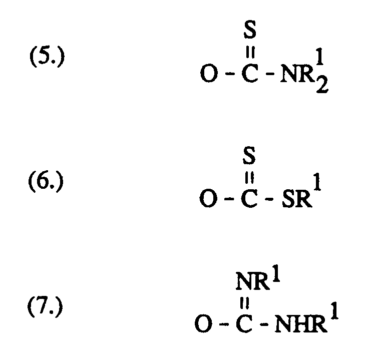
wobei R¹ für C₁-C₁₂-Alkyl, vorzugsweise Methyl, C₆-C₁₂-Aryl, vorzugsweise Phenyl oder Tolyl, bzw. im Fall (3.) Phenylsulfonyl steht, 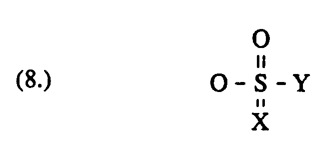
wobei
X = ein freies Elektronenpaar, Y = R¹ oder
X = ein freies Elektronenpaar, Y = OR¹ oder
X = ein freies Elektronenpaar, Y = Cl oder
X = O, Y = R¹ oder
X = O, Y = OR¹ oder
X = O, Y = Cl,
und R¹ = C₁-C₄-Alkyl, Phenyl oder Tolyl bedeuten, 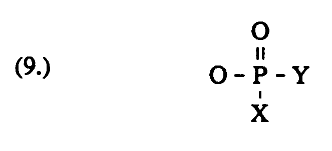
wobei
X = OR¹, Y = Cl oder
X = OR¹, Y = OR¹ oder
X = Cl, Y = Cl
und R¹ = C₁-C₄-Alkyl, Phenyl oder Tolyl bedeuten,
ausgewählt ist.

Die erfindungsgemäßen Verbindungen (IX) eröffnen als Zwischenprodukte für die Herstellung von 15-Pentadecanolid (I) und seinen Homologen eine elegante Synthesemöglichkeit, nach der sich hohe Ausbeuten erzielen lassen.

Weiterer Gegenstand der Erfindung ist ein Verfahren zur Herstellung der Verbindungen (IX), wonach man das Hydroxylacton (VIIa) mit einem geeigneten, vorzugsweise käuflichen Reagenz umsetzt. Bevorzugte solche Reagenzien umfassen beispielsweise
(1.) Carbonsäureanhydride oder -chloride, vorzugsweise Acetanhydrid, Propionsäureanhydrid, Benzoylchlorid, Perlargonsäurechlorid,
(2.) Chlorameisensäureester, vorzugsweise Chlorameisensäuremethylester,
(3.) Alkyl- und/oder Arylisocyanate bzw. Alkyl- und/oder Arylsulfonylisocyanate, vorzugsweise Phenylisocyanat und Phenylsulfonylisocyanat,
(4.) Chlorthioameisensäureester, vorzugsweise Chlorthioameisensäurephenylester,
(5.) Alkyl- und/oder Arylisothiocyanate, vorzugsweise Phenylisothiocyanat,
(6.) Schwefelkohlenstoff (und anschließende S-Alkylierung mit) Methyliodid,
(7.) Dialkyl- und/oder Diarylcarbodiimide, vorzugsweise Dicyclohexylcarbodiimid,
(8.) Schwefelsäurechloride oder Alkylschwefelsäurechloride, vorzugsweise Methansulfonsäurechlorid,
(9.) Phosphorsäurechloride, bevorzugt Diethylchlorphosphat.

Der Begriff "Alkyl" umfaßt im Sinne der vorliegenden Erfindung auch "Cycloalkyl".

Die Umsetzung erfolgt im allgemeinen unter Verwendung von 0,9 bis 2,0, vorzugsweise 1,0 bis 1,5 Mol Reagenz pro Mol Hydroxylacton (VIIa) bei Temperaturen von -5°C bis 150°C, vorzugsweise 0°C bis 135°C. Geeignete Katalysatoren können in üblicher Weise eingesetzt werden.

Weiterer Gegenstand der Erfindung ist ein Verfahren zur Herstellung der ungesättigten Lactone (VIIIa) durch Pyrolyse der Verbindungen IX.

Die Pyrolyse kann bei 110°C bis 450°C, vorzugsweise bei 130°C bis 200°C durchgeführt werden.

Weiterer Gegenstand der Erfindung ist ein Verfahren zur Herstellung von gesättigten Lactonen (Ia) durch Überführung des Hydroxylactons (VIIa) in die Verbindung IX, Pyrolyse von IX zu VIIIa und anschließende Hydrierung.

Die Prozentangaben der nachfolgenden Beispiele beziehen sich auf das Gewicht.

### Beispiele

### A. Herstellung der Ausgangsprodukte

### 1. Herstellung von 12-Oxo-15-pentadecanolid (VI)

219 g (2,45 Mol) Isopropylnitrit, 880 g Isopropanol, 1 000 g Wasser und 79 g 10 %ige Salzsäure werden in einem Kolben vorgelegt und auf 0°C gekühlt. Zu dieser Mischung tropft man 500 g (2,16 Mol) Decamethylendthydropyran (II) (96 %ig) so zu, daß die Innentemperatur nicht über 5°C steigt. Nach dem Zutropfen läßt man noch 2 Stunden bei der gleichen Temperatur nachreagieren. Dem Reaktionsgemisch werden nun 600 g (5,8 Mol) Natriumhydrogensulfit zugemischt und die Mischung bis zum Rückfluß aufgeheizt. Es wird 2,5 Stunden am Rückfluß gekocht. Die organische Phase wird nach Extraktion in Toluol und Bicarbonatwäsche getrocknet, das Lösungsmittel abgezogen und der Rückstand fraktioniert destilliert. Man erhält 480 g (1,89 Mol) 12-Oxo-15-pentadecanolid;
Siedepunkt; 145°C bei 0,5 Torr, Schmelzpunkt: 33°C, Ausbeute 88 % der Theorie.

### 2. Herstellung von 12-Hydroxy-15-pentadecanolid (VII) (Methode Reduktion mit NaBH₄)

Eine Lösung von 480 g (1,89 Mol) 12-Oxo-15-pentadecanolid (VI) in 735 ml Methanol wird auf 10-15°C gekühlt. Bei dieser Temperatur werden portionsweise 26,2 g Natriumborhydrid zugegeben. Man läßt noch 2 Stunden bei 15°C nachreagieren. Nach Zugabe von Wasser wird überschüssiges Methanol abdestilliert. Der organische Anteil des Reaktionsgemisches wird mit Toluol extrahiert, nach Neutralwäsche und Trocknung der toluolischen Phase das Toluol abgezogen und der Rückstand frationiert destilliert. Man erhält 470 g (1,85 Mol) 12-Hydroxy-15-pentadecanolid;
Siedepunkt: 165-172°C bei 2 Torr, Schmelzpunkt: 59-61°C, Ausbeute 97 % der Theorie.

### 3. Herstellung von 12-Hydroxy-15-pentadecanolid (VII) (Methode Hydrierung)

480 g (1,89 Mol) 12-Oxo-15-pentadecanolid (VI) werden mit 1 000 g Isopropanol vermischt und im Autoklaven unter Zugabe von 9,6 g Raney-Kobalt bei 100 bar Wasserstoffdruck und 135°C hydriert. Nach Abziehen des Lösungsmittels erhält man durch Destillation des Rückstands 460 g (1,80 Mol) 12-Hydroxy-15-pentadecanolid.
Ausbeute: 95 % der Theorie.

### B. Herstellung der Derivate IX

### 1. Herstellung von 12-Acetoxy-15-pentadecanolid

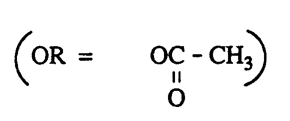

In einem Kolben werden 473 g (4,64 Mol) Essigsäureanhydrid vorgelegt und auf 135-140°C (Rückfluß) erhitzt. Dazu werden 982 g (3,83 Mol) 12-Hydroxy-15-pentadecanolid (VII) getropft. Man läßt noch 1 Stunde unter Rückfluß nachreagieren. Danach wird der Überschuß Essigsäure/Essigsäureanhdyrid im schwachen Vakuum abdestilliert. Man erhält 1120 g 12-Acetoxy-15-pentadecanolid;
Ausbeute: 98 % der Theorie.

### 2. Herstellung von 12-Methoxycarbonyloxy-15-pentadecanolid

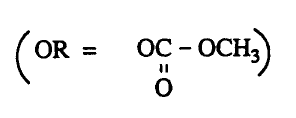

480 g (1,89 Mol) 12-Hydroxy-15-pentadecanolid (VII) werden mit 205,5 g Pyridin in 900 ml Methylenchlorid gelöst. Zu dieser Mischung werden bei 0 bis 5°C innerhalb 1 Stunde 213 g (2,2 Mol) Chlorameisensäuremethylester in 900 ml Methylenchlorid zugetropft. Das Reaktionsgemisch wird weitere 5 Stunden bei Raumtemperatur gerührt. Die organische Phase wird neutral gewaschen und über Na-sulfat getrocknet. Die von Methylenchlorid befreite organische Phase wird fraktioniert destilliert. Man erhält 540 g 12-Methoxycarbonyloxy-15-pentadecanolid; Ausbeute: 91 % der Theorie.

### 3. Herstellung von 12-N-Phenylcarbamoyloxy-15-pentadecanolid

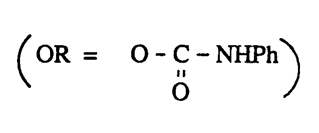

Zu 256 g (1 Mol) VII in 600 ml Methylenchlorid werden 143 g (1,2 Mol) Phenylisocyanat gegeben und anschließend 3 Stunden auf Rückfluß erhitzt. Nach Entfernen des Lösungsmittels liegen ca. 400 g Rohprodukt vor;
Ausbeute: 95 % der Theorie.

### 4. Herstellung von 12-Phenoxythiocarbonyloxy-15-pentadecanolid

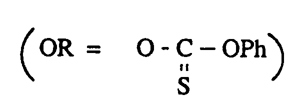

Die Herstellung erfolgt analog zu dem Beispiel B2 mit Chlorthioameisensäurephenylester;
Ausbeute: 89 % der Theorie.

### 5. Herstellung von 12-N-Phenylthiocarbamoyloxy-15-pentadecanolid

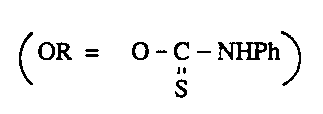

Die Herstellung erfolgt analog zu dem Beispiel B3 mit Phenylisothlocyanat:
Ausbeute: 90 % der Theorie.

### 6. Herstellung von 12-Methylthio-thiocarbonyloxy-15-pentadecanolid

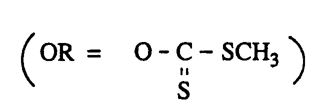

256 g (1,0 Mol) VII in 1 000 ml Toluol werden portionsweise mit insgesamt 33 g (1,1 Mol) Natriumhydrid bei Raumtemperatur versetzt und 24 Stunden auf 80°C erhitzt. Nach Abkühlen gibt man 84 g (1,1 Mol) Schwefelkohlenstoff zu, rückflussiert 24 Stunden, kühlt ab, gibt dann 170 g (1,2 Mol) Methyliodid zu und rückflussiert wiederum 24 Stunden. Nach Hydrolyse und mehrmaliger Wäsche mit Wasser zieht man das Lösungsmittel ab, wonach ca. 380 g Rohprodukt vorliegen;
Ausbeute: 90 % der Theorie.

### 7. Herstellung von 12-(N,N'-Dicyclohexylamidino-oxy)-15-pentadecanolid

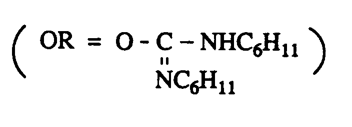

256 g (1,0 Mol) VII, 206 g (1,0 Mol) Dicyclohexylcarbodiimid und 1 g Kupfer-(I)-chlorid in 500 ml Ether werden 72 Stunden bei Raumtemperatur gerührt. Nach Abziehen des Lösungsmittels liegen ca 460 g Rohprodukt vor;
Ausbeute: 94 % der Theorie.

### 8. Herstellung von 12-Mesyloxy-15-pentadecanolid

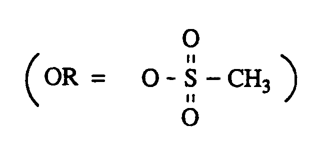

Eine Losung von 256 g VII in Toluol wird in Anwesenheit einer äquimolaren Menge Dimethylbenzylamin mit 127 g Methansulfonsäurechlorid bei einer Temperatur von 30°C versetzt und 1 Stunde gerührt. Nach Waschen der organischen Phase und Abziehen des Lösungsmittels erhält man das Rohprodukt;
Ausbeute: 95 % der Theorie.

### 9. Herstellung von 12-Diethoxyphosphinyloxy-15-pentadecanolid

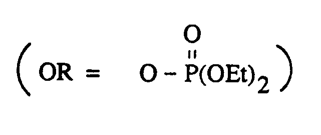

Zu einer Suspension von 33 g (1,1 Mol) Na-hydrid in 300 ml Ether wird bei 0°C eine Lösung von 256 g (1,0 Mol) VII in 500 ml Ether getropft und 1 Stunde bei Raumtemperatur gerührt. Anschließend erfolgt die Zugabe von 173 g (1,0 Mol) Diethylchlorophosphat in 200 ml Ether bei 0°C, wonach noch 2 Stunden bei Raumtemperatur gerührt wird. Nach Hydrolyse und Abziehen des Lösungsmittels erhält man das Rohprodukt;
Ausbeute: 90 % der Theorie.

### C. Pyrolyse

1. (a) 1 120 g (3,75 Mol) 12-Acetoxy-15-pentadecanolid aus Beispiel B1 werden durch ein auf 430°C geheiztes Pyrolyserohr, welches mit Glaskörpern gefüllt ist, getropft. Das Pyrolysat wird mit Toluol extrahiert und der Extrakt mit Wasser und wäßriger Natriumbicarbonatlösung gewaschen. Nach Destillation von Toluol erhält man bei 110-140°C/0,5 Torr eine Hauptfraktion von 652 g, die laut Gaschromatogramm zu 78 % aus einem Gemisch der 15-Pentadecenolide besteht; Ausbeute: 85 % der Theorie.
   (b) Im nachfolgenden Beispiel wird durch Verwendung des Katalysators Zinkchlorid die Reaktionstemperatur (Acetatabspaltung) auf 135-180°C gesenkt (sonst analog zu 1a).
   471 g (1,58 Mol) 12-Acetoxypentadecanolid werden in einem Kolben mit 47 g Zinkchlorid versetzt. Unter Wasserstrahlvakuum (20 Torr) wird beim Erhitzen auf 135-180°C Essigsäure abgespalten, die bei einer Kopftemperatur von 50-60°C abdestilliert wird.
   Die Reaktion ist beendet, wenn keine Essigsäure mehr abgespalten wird (3 bis 5 Stunden). Der verbleibende Rückstand wird nach Extraktion mit Toluol und Neutralwäsche sowie Trocknung im Hochvakuum destilliert. Man erhält 341 g, die zu 96 % aus den isomeren 15-Pentadecenoliden bestehen; Ausbeute: 87 % der Theorie.
2. Das 12-Methoxycarbonyloxy-15-pentadecanolid aus Beispiel B2 wird analog der Pyrolyse gemäß Beispiel C 1a umgesetzt, wobei die Temperatur auf 350-400°C gehalten wird; Ausbeute: 86 % der Theorie.
3. Das rohe Carbamat aus Beispiel B3 wird 3 Stunden auf 200°C erhitzt und nach Abkühlen in Toluol aufgenommen. Nach Waschen mit Wasser und Entfernen des Lösungsmittels wird das Gemisch der 15-Pentadecenolide überdestilliert; Ausbeute: 90 % der Theorie.
4. Die Umsetzung des Derivats aus Beispiel B4 erfolgt analog zu C2; Ausbeute: 80 % der Theorie.
5. Die Pyrolyse des Thiocarbamats aus Beispiel B5 wird analog zu C3 durchgeführt; Ausbeute: 85 % der Theorie.
6. Das rohe Xanthogenat aus Beispiel B6 wird der Pyrolyse gemäß C3 unterzogen; Ausbeute: 80 % der Theorie.
7. Das Rohprodukt aus Beispiel B7 wird auf 200°C/5 mbar erhitzt, wobei das entstehende Gemisch aus den 15-Pentadecenoliden kontinuierlich überdestilliert wird; Ausbeute: 80 % der Theorie.
8. Das Mesyloxy-Derivat aus Beispiel B8 wird in Anwesenheit einer äquimolaren Menge Dimethylbenzylamin 1 Stunde auf 160°C erhitzt. Nach Abkühlen auf Raumtemperatur, Waschen mit Wasser und Destillation erhält man die 15-Pentadecenolide; Ausbeute: 95 % der Theorie.
9. Das rohe Phosphat aus Beispiel B9 wird analog zu der Pyrolyse C8 behandelt; Ausbeute: 90 % der Theorie.

### D. Hydrierung

652 g des Produkts aus Beispiel C1 werden im Autoklaven mit 1 000 ml Methanol und 32,6 g Raney-Nickel vermischt und bei einem Druck von 20 bar und 20°C mit Wasserstoff hydriert. Nach Aufarbeiten erhält man durch fraktionierte Destillation 488 g Pentadecanolid (I); Siedepunkt: 110-114°C/0,5 Torr; Ausbeute: 95,3 % der Theorie.

## Patentansprüche

1. Lactone der Formel 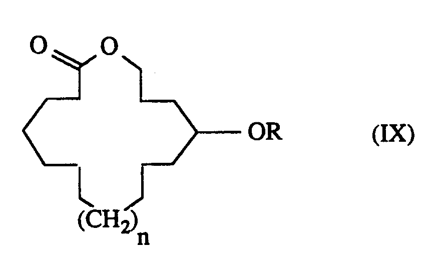 worin
n Null, 1 oder 2 bedeutet und
OR aus der Reihe bestehend aus 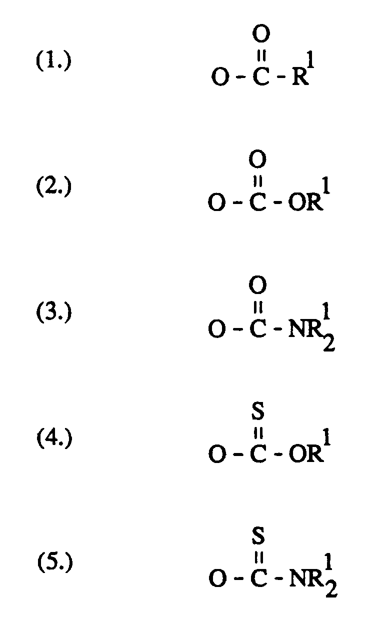 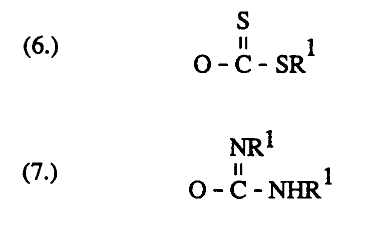 wobei R¹ für C₁-C₁₂-Alkyl, C₆-C₁₂-Aryl, bzw. im Fall (3.) Phenylsulfonyl steht, 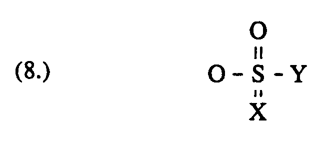 wobei
X = ein freies Elektronenpaar, Y = R¹ oder
X = ein freies Elektronenpaar, Y = OR¹ oder
X = ein freies Elektronenpaar, Y = Cl oder
X = O, Y = R¹ oder
X = O, Y = OR¹ oder
X = O, Y = Cl,
und R¹ = C₁-C₄-Alkyl, Phenyl oder Tolyl bedeuten, 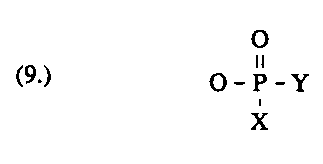 wobei
X = OR¹, Y = Cl oder
X = OR¹, Y = OR¹ oder
X = Cl, Y = Cl
und R¹ = C₁-C₄-Alkyl, Phenyl oder Tolyl bedeuten,
ausgewählt ist.

2. Lactone nach Anspruch 1, worin n 1 bedeutet und OR aus der Reihe Mesyl, Tosyl, Acetoxy ausgewählt ist.

3. Verfahren zur Herstellung der Lactone nach Ansprüchen 1 und 2, wonach man das Hydroxylacton 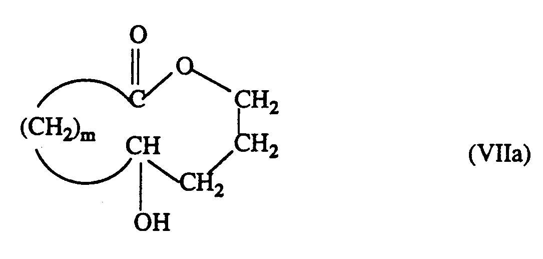 m = 9, 10 oder 11
mit einem geeigneten Reagenz umsetzt.

4. Verfahren zur Herstellung von ungesättigten Lactonen 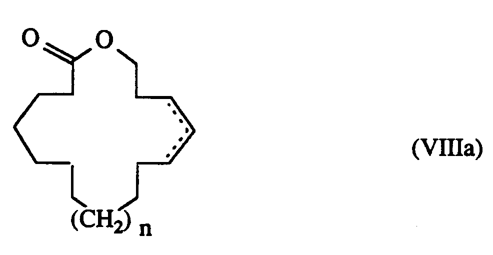 n = Null, 1 oder 2
durch Pyrolyse von Lactonen nach Ansprüchen 1 und 2.

5. Verfahren zur Herstellung von Lactonen der Formel 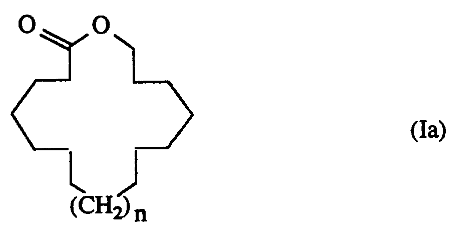 n = Null, 1 oder 2
durch Überführung von Hydroxylacton VIIa in ein Lacton nach Ansprüchen 1 und 2, Pyrolyse des erhaltenen Lactons und Hydrierung.

## Claims

1. Lactones of the formula 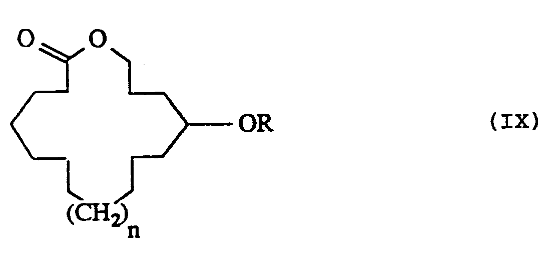 wherein
n denotes zero, 1 or 2 and
OR is chosen from the series consisting of 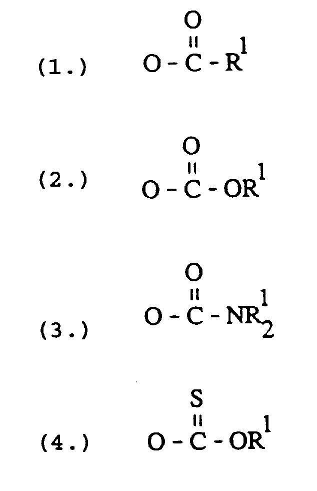 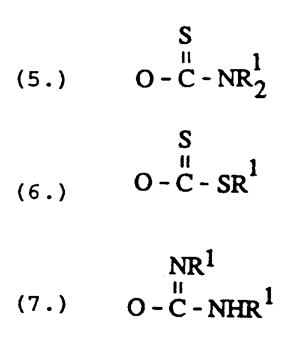 wherein R¹ represents C₁-C₁₂-alkyl, C₆-C₁₂-aryl or, in the case of (3.), phenylsulphonyl, 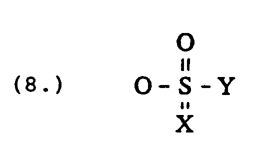 wherein
X = a free electron pair and Y = R¹, or
X = a free electron pair and Y = OR¹, or
X = a free electron pair and Y = Cl, or
X = O and Y = R¹, or
X = O and Y = OR¹, or
X = O and Y = Cl,
and R¹ denotes C₁-C₄-alkyl, phenyl or tolyl, and 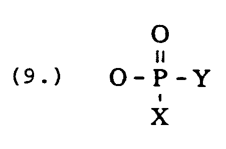 wherein
X = OR¹ and Y = Cl, or
X = OR¹ and Y = OR¹, or
X = Cl and Y = Cl,
and R¹ denotes C₁-C₄-alkyl, phenyl or tolyl.

2. Lactones according to Claim 1, wherein n denotes 1 and OR is chosen from the series comprising mesyl, tosyl and acetoxy.

3. Process for the preparation of the lactones according to Claims 1 and 2, in which the hydroxylactone 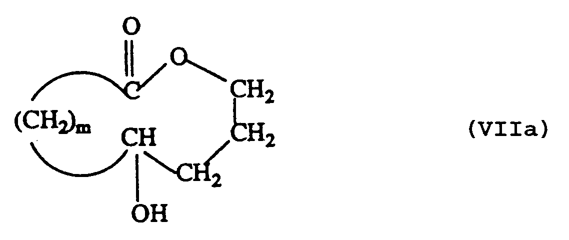 m = 9, 10 or 11
is reacted with a suitable reagent.

4. Process for the preparation of unsaturated lactones 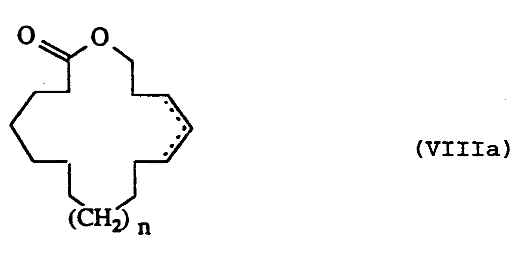 n = zero, 1 or 2
by pyrolysis of lactones according to Claims 1 and 2.

5. Process for the preparation of lactones of the formula 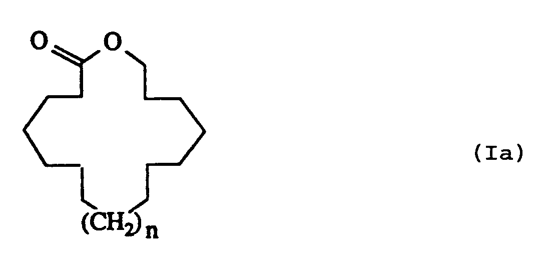 n = zero, 1 or 2
by conversion of hydroxylactone VIIa into a lactone according to Claims 1 and 2, pyrolysis of the resulting lactone and hydrogenation.

## Revendications

1. Lactones de formule 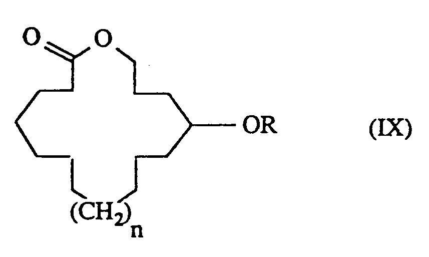 dans laquelle
n est égal à 0, 1 ou 2 et
OR est choisi parmi 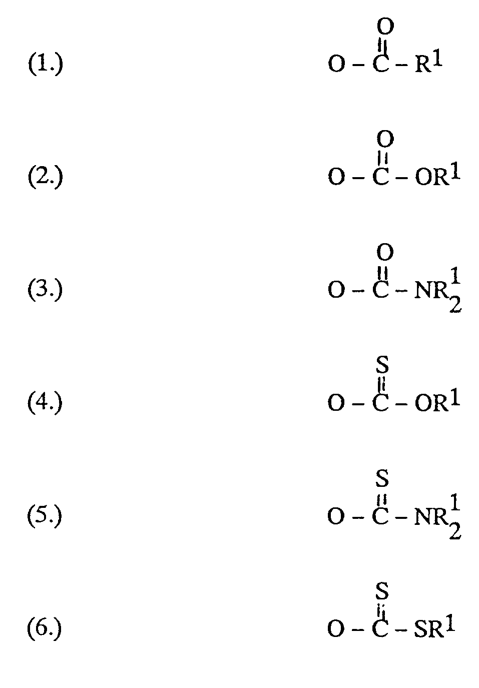 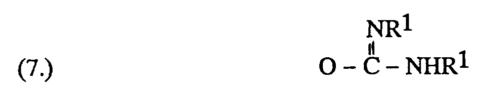 dans lesquels R1 représente un groupe alkyle en C₁-C₁₂, aryle en C₆-C₁₂ ou bien, dans le cas (3.) un groupe phénylsulfonyle, 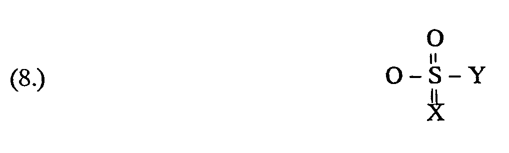 avec
X = une paire d'électrons libres, Y = R¹ ou
X = une paire d'électrons libres Y = OR¹ ou
X = une paire d'électrons libres Y = Cl ou
X = O, Y = R¹ ou
X = O, Y = OR¹ ou
X = O, Y = Cl,
et R¹ = alkyle en C₁-C₄, phényle ou tolyle, 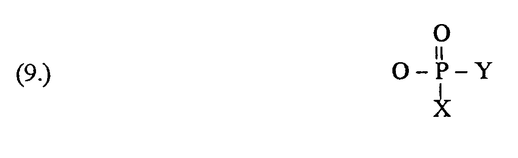 avec
X = OR¹, Y = Cl ou
X = OR¹, Y = OR¹ ou
X = Cl, Y = Cl
et R¹ = alkyle en C₁-C₄, phényle ou tolyle.

2. Lactones selon revendication 1, pour lesquelles n = 1 et OR est choisi parmi les groupes méthanesulfonyle, toluènesulfonyle, acétoxy.

3. Procédé de préparation des lactones selon revendications 1 et 2, selon lequel on fait réagir l'hydroxylactone 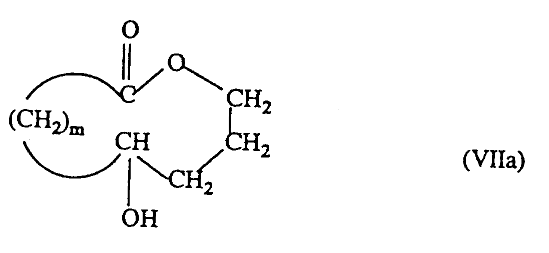 m = 9, 10 ou 11
avec un réactif approprié.

4. Procédé de préparation des lactones insaturées 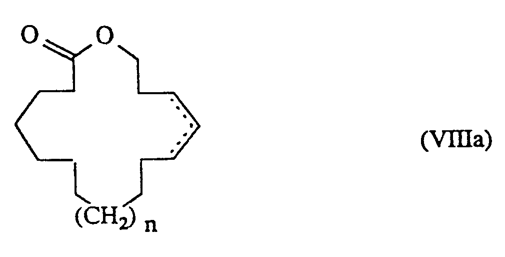 n = 0, 1 ou 2
par pyrolyse des lactones selon revendications 1 et 2.

5. Procédé de préparation des lactones de formule 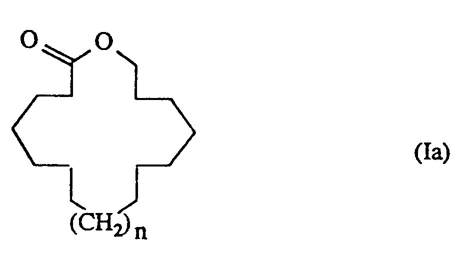 n = 0, 1 ou 2
par conversion de l'hydroxylactone VIIa en une lactone selon les revendications 1 et 2, pyrolyse de la lactone obtenue et hydrogénation.
